# EUROPÄISCHE PATENTSCHRIFT

(11) **EP 2 437 747 B1**
(45) Veröffentlichungstag und Bekanntmachung des Hinweises auf die Patenterteilung: **31.08.2016**
(21) Anmeldenummer: 10800713.9
(22) Anmeldetag: 10.12.2010
(51) Int. Cl.: A61K 31/573, A61K 38/17, A61K 38/20, A61K 45/06, A61P 19/02, A61P 19/00, A61P 37/00, A61K 38/00, A61K 9/00, A61K 9/51, A61K 35/15, A61K 35/19

(54) **KOMBINATIONSPRÄPARATE MIT EXOSOMEN UND CORTICOSTEROID**
COMBINATION PREPARATION COMPRISING AN EXOSOME AND A CORTICOSTEROID
PRÉPARATION COMBINÉE CONTENANT EXOSOMES ET UN CORTICOSTÉROÏDE

(30) Priorität: 10.12.2009 DE 102009057495
(43) Veröffentlichungstag der Anmeldung: 11.04.2012
(73) Patentinhaber: Orthogen AG, 40210 Düsseldorf (DE)
(72) Erfinder: WEHLING, Peter, 50597 Düsseldorf (DE); REINECKE, Julio, 50733 Köln (DE)
(74) Vertreter: König, Gregor Sebastian
(86) Internationale Anmeldenummer: PCT/EP2010/069426
(87) Internationale Veröffentlichungsnummer: WO 2011/082950

(56) Entgegenhaltungen:
- WO-A2-2006/007529
- Dr med F.-U. Hornstein: arznei-telegramm, Bd. 32, Nr. 3 2001, Seiten 34-34, XP002629197, Gefunden im Internet: URL:http://www.arznei-telegramm.de/registe r/0103034.pdf [gefunden am 2011-03-21]
- BIANCO NICOLE R ET AL: "Therapeutic Effect of Exosomes From Indoleamine 2,3-Dioxygenase-Positive Dendritic Cells in Collagen-Induced Arthritis and Delayed-Type Hypersensitivity Disease Models", ARTHRITIS & RHEUMATISM, Bd. 60, Nr. 2, Februar 2009 (2009-02), Seiten 380-389, XP002628890, ISSN: 0004-3591
- BRESNIHAN BARRY ET AL: "Treatment of rheumatoid arthritis with recombinant human interleukin-1 receptor antagonist", ARTHRITIS AND RHEUMATISM, Bd. 41, Nr. 12, Dezember 1998 (1998-12), Seiten 2196-2204, XP009146178, ISSN: 0004-3591
- SETTAS LUCAS D ET AL: "Reactivation of pulmonary tuberculosis in a patient with rheumatoid arthritis during treatment with IL-1 receptor antagonists (anakinra).", JOURNAL OF CLINICAL RHEUMATOLOGY : PRACTICAL REPORTS ON RHEUMATIC & MUSCULOSKELETAL DISEASES AUG 2007 LNKD- PUBMED:17762459, Bd. 13, Nr. 4, August 2007 (2007-08), Seiten 219-220, XP009146179, ISSN: 1076-1608

### SEQUENZPROTOKOLL

<110> Orthogen AG
<120> Kombinationspräparate mit Corticosteroid und Exosomen
<130> 52 253 K
<160> 8
<170> PatentIn version 3.3
<210> 1
   <211> 177
   <212> PRT
   <213> Homo sapiens
<400> 1
   Met Glu Ile Cys Arg Gly Leu Arg Ser His Leu Ile Thr Leu Leu Leu
   1               5                   10                  15
   Phe Leu Phe His Ser Glu Thr Ile Cys Arg Pro Ser Gly Arg Lys Ser
               20                  25                  30
   Ser Lys Met Gln Ala Phe Arg Ile Trp Asp Val Asn Gln Lys Thr Phe
           35                  40                  45
   Tyr Leu Arg Asn Asn Gln Leu Val Ala Gly Tyr Leu Gln Gly Pro Asn
       50                  55                  60
   Val Asn Leu Glu Glu Lys Ile Asp Val Val Pro Ile Glu Pro His Ala
   65                  70                  75                  80
   Leu Phe Leu Gly Ile His Gly Gly Lys Met Cys Leu Ser Cys Val Lys
                   85                  90                  95
   Ser Gly Asp Glu Thr Arg Leu Gln Leu Glu Ala Val Asn Ile Thr Asp
               100                 105                 110
   Leu Ser Glu Asn Arg Lys Gln Asp Lys Arg Phe Ala Phe Ile Arg Ser
           115                 120                 125
   Asp Ser Gly Pro Thr Thr Ser Phe Glu Ser Ala Ala Cys Pro Gly Trp
       130                 135                 140
   Phe Leu Cys Thr Ala Met Glu Ala Asp Gln Pro Val Ser Leu Thr Asn
   145                 150                 155                 160
   Met Pro Asp Glu Gly Val Met Val Thr Lys Phe Tyr Phe Gln Glu Asp
                   165                 170                 175
   Glu
<210> 2
   <211> 159
   <212> PRT
   <213> Homo sapiens
<400> 2
   Met Ala Leu Glu Thr Ile Cys Arg Pro Ser Gly Arg Lys Ser Ser Lys
   1               5                   10                  15
   Met Gln Ala Phe Arg Ile Trp Asp Val Asn Gln Lys Thr Phe Tyr Leu
               20                  25                  30
   Arg Asn Asn Gln Leu Val Ala Gly Tyr Leu Gln Gly Pro Asn Val Asn
           35                  40                  45
   Leu Glu Glu Lys Ile Asp Val Val Pro Ile Glu Pro His Ala Leu Phe
       50                  55                  60
   Leu Gly Ile His Gly Gly Lys Met Cys Leu Ser Cys Val Lys Ser Gly
   65                  70                  75                  80
   Asp Glu Thr Arg Leu Gln Leu Glu Ala Val Asn Ile Thr Asp Leu Ser
                   85                  90                  95
   Glu Asn Arg Lys Gln Asp Lys Arg Phe Ala Phe Ile Arg Ser Asp Ser
               100                 105                 110
   Gly Pro Thr Thr Ser Phe Glu Ser Ala Ala Cys Pro Gly Trp Phe Leu
           115                 120                 125
   Cys Thr Ala Met Glu Ala Asp Gln Pro Val Ser Leu Thr Asn Met Pro
       130                 135                 140
   Asp Glu Gly Val Met Val Thr Lys Phe Tyr Phe Gln Glu Asp Glu
   145                 150                 155
<210> 3
   <211> 180
   <212> PRT
   <213> Homo sapiens
<400> 3
   Met Ala Leu Ala Asp Leu Tyr Glu Glu Gly Gly Gly Gly Gly Gly Glu
   1               5                   10                  15
   Gly Glu Asp Asn Ala Asp Ser Lys Glu Thr Ile Cys Arg Pro Ser Gly
               20                  25                  30
   Arg Lys Ser Ser Lys Met Gln Ala Phe Arg Ile Trp Asp Val Asn Gln
               35                  40                  45
   Lys Thr Phe Tyr Leu Arg Asn Asn Gln Leu Val Ala Gly Tyr Leu Gln
       50                  55                  60
   Gly Pro Asn Val Asn Leu Glu Glu Lys Ile Asp Val Val Pro Ile Glu
   65                  70                  75                  80
   Pro His Ala Leu Phe Leu Gly Ile His Gly Gly Lys Met Cys Leu Ser
                   85                  90                  95
   Cys Val Lys Ser Gly Asp Glu Thr Arg Leu Gln Leu Glu Ala Val Asn
               100                 105                 110
   Ile Thr Asp Leu Ser Glu Asn Arg Lys Gln Asp Lys Arg Phe Ala Phe
           115                 120                 125
   Ile Arg Ser Asp Ser Gly Pro Thr Thr Ser Phe Glu Ser Ala Ala Cys
       130                 135                 140
   Pro Gly Trp Phe Leu Cys Thr Ala Met Glu Ala Asp Gln Pro Val Ser
   145                 150                 155                 160
   Leu Thr Asn Met Pro Asp Glu Gly Val Met Val Thr Lys Phe Tyr Phe
                   165                 170                 175
   Gln Glu Asp Glu
               180
<210> 4
   <211> 143
   <212> PRT
   <213> Homo sapiens
<400> 4
   Met Gln Ala Phe Arg Ile Trp Asp Val Asn Gln Lys Thr Phe Tyr Leu
   1               5                   10                  15
   Arg Asn Asn Gln Leu Val Ala Gly Tyr Leu Gln Gly Pro Asn Val Asn
               20                  25                  30
   Leu Glu Glu Lys Ile Asp Val Val Pro Ile Glu Pro His Ala Leu Phe
           35                  40                  45
   Leu Gly Ile His Gly Gly Lys Met Cys Leu Ser Cys Val Lys Ser Gly
       50                  55                  60
   Asp Glu Thr Arg Leu Gln Leu Glu Ala Val Asn Ile Thr Asp Leu Ser
   65                  70                  75                  80
   Glu Asn Arg Lys Gln Asp Lys Arg Phe Ala Phe Ile Arg Ser Asp Ser
                   85                  90                  95
   Gly Pro Thr Thr Ser Phe Glu Ser Ala Ala Cys Pro Gly Trp Phe Leu
               100                 105                 110
   Cys Thr Ala Met Glu Ala Asp Gln Pro Val Ser Leu Thr Asn Met Pro
           115                 120                 125
   Asp Glu Gly Val Met Val Thr Lys Phe Tyr Phe Gln Glu Asp Glu
       130                 135                 140
<210> 5
   <211> 155
   <212> PRT
   <213> Homo sapiens
<400> 5
<211> 177
   <212> PRT
   <213> Equus caballus
<400> 6
<210> 7
   <211> 177
   <212> PRT
   <213> Equus caballus
<400> 7
   Met Glu Ile Arg Arg Arg Ser Val Arg His Leu Ile Ser Leu Leu Leu
   1               5                   10                  15
   Phe Leu Leu Tyr Ser Glu Thr Ala Cys His Pro Leu Gly Lys Arg Pro
               20                  25                  30
   Cys Lys Met Gln Ala Phe Arg Ile Trp Asp Val Asn Gln Lys Thr Phe
           35                  40                  45
   Tyr Met Arg Asn Asn Gln Leu Val Ala Gly Tyr Leu Gln Glu Ser Asn
       50                  55                  60
   Thr Lys Leu Gln Glu Lys Ile Asp Val Val Pro Ile Glu Pro Asp Ala
   65                  70                  75                  80
   Leu Phe Leu Gly Leu His Gly Arg Lys Leu Cys Leu Ala Cys Val Lys
                   85                  90                  95
   Ser Gly Asp Glu Ile Arg Phe Gln Leu Glu Ala Val Asn Ile Thr Asp
               100                 105                 110
   Leu Ser Lys Asn Lys Glu Glu Asn Lys Arg Phe Thr Phe Ile Arg Ser
           115                 120                 125
   Asn Ser Gly Pro Thr Thr Ser Phe Glu Ser Ala Ala Cys Pro Gly Trp
       130                 135                 140
   Phe Leu Cys Thr Ala Gln Glu Ala Asp Arg Pro Val Ser Leu Thr Asn
   145                 150                 155                 160
   Lys Pro Lys Glu Ser Phe Met Val Thr Lys Phe Tyr Leu Gln Glu Asp
                   165                 170                 175
   Gln
<210> 8
   <211> 176
   <212> PRT
   <213> Canis familiaris
<400> 8
   Met Glu Thr Cys Arg Cys Pro Leu Ser Tyr Leu Ile Ser Phe Leu Leu
   1               5                   10                  15
   Phe Leu Ser His Ser Glu Thr Ala Cys Arg Pro Leu Gly Lys Arg Pro
               20                  25                  30
   Cys Arg Met Gln Ala Phe Arg Ile Trp Asp Val Asn Gln Lys Thr Phe
           35                  40                  45
   Tyr Leu Arg Asn Asn Gln Leu Val Ala Gly Tyr Leu Gln Gly Ser Asn
       50                  55                  60
   Thr Lys Leu Glu Glu Lys Leu Asp Val Val Pro Val Glu Pro His Ala
   65                  70                  75                  80
   Val Phe Leu Gly Ile His Gly Gly Lys Leu Cys Leu Ala Cys Val Lys
                   85                  90                  95
   Ser Gly Asp Glu Thr Arg Leu Gln Leu Glu Ala Val Asn Ile Thr Asp
               100                 105                 110
   Leu Ser Lys Asn Lys Asp Gln Asp Lys Arg Phe Thr Phe Ile Leu Ser
           115                 120                 125
   Asp Ser Gly Pro Thr Thr Ser Phe Glu Ser Ala Ala Cys Pro Gly Trp
       130                 135                 140
   Phe Leu Cys Thr Ala Leu Glu Ala Asp Arg Pro Val Ser Leu Thr Asn
   145                 150                 155                 160
   Arg Pro Glu Glu Ala Met Met Val Thr Lys Phe Tyr Phe Gln Lys Glu
                   165                 170                 175

## Beschreibung

### Gebiet der Erfindung

Die vorliegende Erfindung betrifft pharmazeutische Zusammensetzungen für eine Kombinationstherapie mit einem Corticosteroid und Exosomen. Mittels der Kombinationstherapie können Krankheiten wie Arthrose, Arthritis und/oder degenerative Wirbelsäulenerkrankungen behandelt werden, wobei die Behandlung vorzugsweise lokal erfolgt.

### Technischer Hintergrund

Arthrose bezeichnet in Deutschland einen "Gelenkverschleiß", der das altersübliche Maß übersteigt. Er geht einher mit Verlust von Knorpel im betroffenen Gelenk, woraus sich Schmerzen und Funktionsverschlechterungen ergeben. Ursächlich werden ein Übermaß an Belastung, angeborene oder traumatisch bedingte Ursachen, wie Fehlstellungen der Gelenke, oder auch knöcherner Deformierung durch Knochenerkrankungen wie Osteoporose gesehen. Sie kann ebenfalls als Folge einer anderen Erkrankung wie etwa Gelenkentzündung entstehen oder mit überlastungsbedingter Ergussbildung einhergehen.

Grundsätzlich können alle Gelenke von arthrotischen Veränderungen betroffen werden. In Deutschland wird die Erkrankung am häufigsten im Kniegelenk lokalisiert. Arthrose ist einer der häufigsten Beratungsanlässe in einer allgemeinmedizinischen Praxis. Etwa 10% der Bevölkerung in westlichen Ländern leidet an Arthrose. Rechnet man die Arthrosen der kleinen Wirbelgelenke und die degenerativen Bandscheibenerkrankungen dazu, sind sogar ca. 15%-20% der Bevölkerung betroffen. Das Risiko an Arthrose zu erkranken, erhöht sich mit zunehmendem Alter. Etwa zwei Drittel der Menschen über 65 Jahren sind von der Erkrankung betroffen, jedoch leiden nicht alle Betroffenen auch an den Symptomen.

Für die Behandlung von Arthrose sind bereits einige Therapieformen bekannt. Hierzu gehören sowohl konservative (z.B. medikamentöse) Therapien als auch chirurgische Eingriffe bis hin zur Ersetzung des vollständigen Gelenks durch eine Prothese. Um solche umfangreichen und unumkehrbaren Eingriffe zu vermeiden, ist grundsätzlich eine wirksame medikamentöse Behandlung vorzuziehen, um den Zeitpunkt eines kompletten Gelenkersatzes möglichst weit herauszuzögern.

Allerdings haben viele medikamentöse Behandlungen auch Nachteile. Zum einen liegt dies an den Nebenwirkungen der Medikamente selbst, aber auch ihre Wirksamkeit ist teilweise nur bedingt gegeben.

Ein oft verwendeter medizinischer Wirkstoff zur Behandlung von Arthrose und rheumatoider Arthritis ist Cortison (auch lokal appliziert) und verwandte Corticosteroide. Diese werden bei RA systemisch und bei Arthrose lokal als Injektion in das betroffene Gelenk verabreicht. Hierbei zeigt sich allerdings, dass die positive Wirkung einer Corticosteroidverabreichung sowohl bei Arthrose als auch Rheumatoider Arthritis bereits nach nur einer Woche deutlich nachlässt. Dies ist klinisch aufgrund randomisierter Studien und klinischer Erfahrungen gesichert. Bei RA versucht man durch eine kontinuierliche Gabe von Cortison systemisch den Wirkspiegel hochzuhalten, allerdings ist dies durch Verstärkung der Nebenwirkungen und durch ein Nachlassen des therapeutischen Effektes bei Dauergabe sehr problematisch.

Exosome sind kleine, von einer Lipidmembran umhüllte Vesikel, die im extrazellulären Raum zum Beispiel des menschlichen Körpers vorkommen. Sie werden von Zellen durch Abspaltung von der zellulären Plasmamembran gebildet und sekretiert. Normalerweise enthalten diese Exosomen auch Proteine, die sie von ihrer Ursprungszelle übernommen haben. Verfahren zur Gewinnung und Verabreichung von Exosomen sind zum Beispiel in der Patentanmeldung WO 2006/007529 A2 beschrieben. Bei der In-vitro-Induktion von prophylaktisch oder therapeutisch wirksamen Proteinen wie etwa IL-1Ra durch Inkubation einer Blutprobe in einem geeigneten Gefäß wie etwa einer Spritze werden Exosomen gebildet. Folglich enthält beispielsweise Orthokin Exosomen. Die Bildung von Exosomen kann durch Hinzufügen eines die Exosomenbildung fördernden Zusatzstoffes gesteigert werden. Die Konzentration der Exosomen kann beispielsweise durch Zentrifugation bei einer hohen Zentrifugalbeschleunigung gesteigert werden. Der Einsatz von Exosomen bei der Behandlung von rheumatoider Arthritis ist an sich bekannt.

In der Veröffentlichung "arznei-telegramm" 2001; Jg. 32, Nr. 3, S. 34 wird der Einsatz von Orthokin bei Arthrose, rheumatoider Arthritis und Bandscheibenvorfall allgemein diskutiert. Nicht offenbart wird ein Corticosteroid oder eine Kombinationstherapie mit einem Corticosteroid und Exosomen.

Bianco et al. (2009) Arthritis & Rheumatism 60, 380 beschäftigt sich mit der Wirkung von Exosomen aus dendritischen Zellen auf verschiedene Mausmodelle von Krankheiten. Bianco et al. betrifft weder aus einer Blutprobe gewonnene Exosomen noch eine Kombinationstherapie mit einem Corticosteroid.

Bresnihan et al. (1998) Arthritis & Rheumatism 41, 2196 offenbart die Behandlung von rheumatoider Arthritis mit rekombinantem humanem IL-1Ra. Ein Teil der Patienten verwendete ein Corticosteroid (Prednisolon), ohne dass eine Wirkung dieser Maßnahme offenbart wäre. Nicht offenbart sind außerdem Exosomen.

Settas et al. (2007) offenbart die Reaktivierung einer Lungen-Tuberkulose bei einem an rheumatoider Arthritis leidenden Patienten, der mit rekombinantem IL-1Ra (Anakinra) behandelt wurde. Er nahm zusätzlich Prednisolon ein, ohne dass eine Wirkung hiervon offenbart wäre. Exosomen sind nicht offenbart.

Ein weiteres Medikament, das zur Behandlung von Arthrose eingesetzt werden kann, ist das körpereigene Protein IL-1Ra oder eine Isoform oder ein Fragment hiervon, welches eine ähnliche Aktivität aufweist. Interleukin-1-Rezeptorantagonist (IL-1Ra) bindet an die selben Rezeptoren auf der Zelloberfläche wie Interleukin-1 (IL-1), löst dort aber nicht die normalerweise durch IL-1-Bindung hervorgerufene Signalkaskade aus. Durch Bindung an den IL-1-Rezeptor blockiert IL-1Ra die Bindung von IL-1 und verhindert so dessen Signalweiterleitung und somit die entzündungsfördernde Wirkung von IL-1 auf die Zielzellen.

Die Behandlung von Patienten mit körpereigenem Serum, in dem IL-1Ra angereichert wurde, ist im Stand der Technik bekannt. Das so eingesetzte IL-1Ra wird auch Orthokin genannt. Ein rekombinantes IL-1Ra-Fragment, Anakinra, zeigte hingegen bei der Behandlung von Arthrosen keinerlei Wirkung im Vergleich zu einer Placebo-Behandlung. Anakinra ist eine auf die Aminosäuren 26 - 177 verkürzte und endständig L-methionylierte Isoform des humanen Interleukin-1-Rezeptorantagonisten und besitzt eine Sequenzlänge von 153 Aminosäuren. Die Herstellung erfolgt zum Beispiel durch *Escherichia coli-*Stämme mit rekombinanten Methoden.

Angesichts des Stands der Technik gab es daher die Aufgabe, eine medikamentöse Arthrose-Behandlung bereitzustellen, die eine bessere Wirksamkeit aufweist und insbesondere eine gute Langzeit-Wirksamkeit besitzt. Die Behandlung sollte zudem vorzugsweise eine gute Wirksamkeit, bevorzugt Langzeit-Wirksamkeit bei Arthritis, insbesondere rheumatoider Arthritis haben.

### Zusammenfassung der Erfindung

Überraschenderweise wurde nun festgestellt, dass die Wirksamkeit, insbesondere die Langzeit-Wirksamkeit und die Schnelligkeit des Wirkungseintritts, von Corticosteroiden wie Cortison bei Arthrose, Arthritis und degenerativer Wirbelsäulenerkrankung durch die zusätzliche Verabreichung von Exosomen deutlich bzw. synergistisch verbessert werden kann. Dies zeigt sich insbesondere bei der lokalen Verabreichung der Therapeutika direkt in das zu behandelnde Gelenk. Eine der überraschenden Erkenntnisse ist etwa, dass die Verwendung von Corticosteroiden in Kombination mit Exosomen, die beispielsweise in einer Orthokin-Spritze aus einer Blutprobe gewonnen wurden (z.B. Corticosteroid zusammen mit Orthokin) im Hinblick auf die Schnelligkeit des Wirkungseintritts bei Arthrose einer Therapie überlegen zu sein scheint, die den Wirkstoff Exosomen nicht beinhaltet. Eine schnell eintretende Wirkung lässt sich auch beobachten, wenn einer an sich bereits wirksamen Arthrosetherapie (etwa Corticosteroid zusammen mit Anakinra) als zusätzlichen Wirkstoff Exosomen hinzufügt.

Eine auffallend gute Wirksamkeit zeigt die Kombination von Corticosteroid und Exosomen auch bei der Behandlung von rheumatoider Arthritis, sowohl was die Schnelligkeit des Wirkungseintritts angeht als auch die Langzeit-Wirksamkeit. Hierbei zeigte sich ein besonders günstiger Effekt bedingt durch überraschend hohe Wirksamkeit (bedingt durch die unterschiedlichen pathophysiologischen Ansatzpunkte) bei besonders günstigem Nebenwirkungsprofil für den Patienten. Das günstige Nebenwirkungsprofil ist von besonderer Bedeutung, da chronische Corticosteroidgaben mit erheblichen Nebenwirkungen wie Stoffwechselentgleisungen, Osteoporose und anderen Nebenwirkungen verknüpft sind. Durch die Kombination von Exosomen mit Corticosteroiden wie Cortison werden aber die ungünstigen Wirkungen der Corticosteroide (des Cortisons) vermindert und die Wirksamkeit der Behandlung erhöht.

Unabhängig von der Indikation hat sich bei etlichen Patienten beobachten lassen, dass die Kombination von Exosomen mit Corticosteroiden einen therapeutischen Effekt zeigt, der über die Summe der einzelnen therapeutischen Effekte der beiden Wirkstoffe Exosome und Corticosteroide hinausgeht, insbesondere wenn nicht beide Wirkstoffe bei vereinzelter Verabreichung therapeutisch wirksam sind.

Daher stellt die vorliegende Erfindung in einem ersten Aspekt eine pharmazeutische Zusammensetzung bereit, die ein Corticosteroid zusammen mit Exosomen in Gegenwart oder Abwesenheit eines Cytokin-Antagonisten und/oder eines Wachstumsfaktors enthält, wobei die Exosomen herstellbar sind durch ein Verfahren umfassend die Schritte: Bereitstellen einer Exosomen enthaltenden Blutprobe und vorzugsweise Aufkonzentrieren der Exosomen.

Der Ausdruck "Abwesenheit eines Cytokin-Antagonisten" bedeutet im Rahmen der vorliegenden Erfindung, dass kein einziger Cytokin-Antagonist anwesend ist. Dies gilt hinsichtlich des Wachstumsfaktors entsprechend.

Die pharmazeutische Zusammensetzung gemäß dem ersten Aspekt enthält also in jedem Fall ein Corticosteroid und Exosomen. Ferner gibt es entweder die Option der Gegenwart oder die Option der Abwesenheit eines Cytokin-Antagonisten; zudem gibt es entweder die Option der Gegenwart oder die Option der Abwesenheit eines Wachstumsfaktors.

Ebenso können die Therapeutika auch in zwei verschiedenen pharmazeutischen Zusammensetzungen gleichzeitig oder in zeitlicher Abfolge verabreicht werden. Entsprechend wird in einem zweiten und einem dritten Aspekt der Erfindung eine pharmazeutische Zusammensetzung enthaltend Exosomen in Gegenwart oder Abwesenheit eines Cytokin-Antagonisten und/oder eines Wachstumsfaktors zur Verwendung in einer Kombinationstherapie zusammen mit einem Corticosteroid, sowie eine pharmazeutische Zusammensetzung enthaltend ein Corticosteroid in Gegenwart oder Abwesenheit eines Cytokin-Antagonisten und/oder eines Wachstumsfaktors zur Verwendung in einer Kombinationstherapie zusammen mit Exosomen bereitgestellt, wobei die Kombinationstherapie in beiden Fällen die Behandlung einer Krankheit betrifft, die ausgewählt aus der Gruppe bestehend aus Gelenkerkrankungen wie Arthrose, Arthritis, Gelenkentzündung und entzündlicher Knorpelverlust, degenerative Wirbelsäulenerkrankungen, Gelenkschmerzen und Autoimmunerkrankungen ist, wobei die Krankheit von rheumatoider Arthritis verschieden ist und die Exosomen aus einer Blutprobe gewonnen wurden.

Die Erfindung stellt in einem vierten Aspekt eine pharmazeutische Zusammensetzung bereit, die einen Cytokin-Antagonisten und/oder einen Wachstumsfaktor zur Verwendung in einer Kombinationstherapie zusammen mit einem Corticosteroid und Exosomen enthält, wobei die Kombinationstherapie die Behandlung einer Krankheit betrifft, die ausgewählt aus der Gruppe bestehend aus Gelenkerkrankungen wie Arthrose, Arthritis, Gelenkentzündung und entzündlicher Knorpelverlust, degenerative Wirbelsäulenerkrankungen, Gelenkschmerzen und Autoimmunerkrankungen ist, wobei die Krankheit von rheumatoider Arthritis verschieden ist und die Exosomen aus einer Blutprobe gewonnen wurden.

In einem fünften erfindungsgemäßen Aspekt wird ein Kit bereitgestellt, der (i) eine pharmazeutische Zusammensetzung enthaltend Exosomen in Gegenwart oder Abwesenheit eines Cytokin-Antagonisten und/oder eines Wachstumsfaktors und (ii) eine pharmazeutische Zusammensetzung enthaltend ein Corticosteroid in Gegenwart oder Abwesenheit eines Cytokin-Antagonisten und/oder eines Wachstumsfaktors enthält, wobei die Exosomen herstellbar sind durch ein Verfahren umfassend die Schritte: Bereitstellen einer Exosomen enthaltenden Blutprobe und vorzugsweise Aufkonzentrieren der Exosomen.

Außerdem betrifft die Erfindung in einem sechsten Aspekt die Verwendung von Exosomen in Gegenwart oder Abwesenheit eines Cytokin-Antagonisten und/oder eines Wachstumsfaktors zur Herstellung einer pharmazeutischen Zusammensetzung zur Verwendung in einer Kombinationstherapie zusammen mit einem Corticosteroid, und in einem siebten Aspekt die Verwendung eines Corticosteroids in Gegenwart oder Abwesenheit eines Cytokin-Antagonisten und/oder eines Wachstumsfaktors zur Herstellung einer pharmazeutischen Zusammensetzung zur Verwendung in einer Kombinationstherapie zusammen mit Exosomen, wobei die Kombinationstherapie in beiden Fällen die Behandlung einer Krankheit betrifft, die ausgewählt aus der Gruppe bestehend aus Gelenkerkrankungen wie Arthrose, Arthritis, Gelenkentzündung und entzündlicher Knorpelverlust, degenerative Wirbelsäulenerkrankungen, Gelenkschmerzen und Autoimmunerkrankungen ist, wobei die Krankheit von rheumatoider Arthritis verschieden ist und die Exosomen aus einer Blutprobe gewonnen wurden.

Die oben im Zusammenhang mit dem zweiten, dritten, vierten, sechsten und siebten Aspekt erwähnte Kombinationstherapie ist vorzugsweise eine Kombinationstherapie zusammen mit einem Cytokin-Antagonisten und/oder einem Wachstumsfaktor, insbesondere in den Fällen, in denen in der pharmazeutischen Zusammensetzung selbst ein Cytokin-Antagonist und/oder ein Wachstumsfaktor abwesend ist.

In einem achten Aspekt betrifft die vorliegende Erfindung ein Verfahren zur Herstellung einer ein Corticosteroid und Exosomen enthaltenden pharmazeutischen Zusammensetzung, umfassend die Schritte: Bereitstellen einer Exosomen enthaltenden Blutprobe, vorzugsweise Aufkonzentrieren der Exosomen und Vermischen mit einem Corticosteroid.

Weitere Ausführungsformen der Erfindung sind in der nachfolgenden ausführlichen Beschreibung und in den Patentansprüchen angegeben.

### Beschreibung der Erfindung

Die Erfindung basiert auf der überraschenden Erkenntnis, dass die Behandlung von Erkrankungen der Gelenke und der Wirbelsäule wie Arthrose, Arthritis und degenerative Wirbelsäulenerkrankung, sowie bei Autoimmunerkrankungen wie Neurodermitis und kreisrunder Haarausfall mittels Corticosteroiden durch die zusätzliche Verabreichung von Exosomen deutlich verbessert werden kann. Somit richtet sich die Erfindung auf die Kombinationstherapie solcher Erkrankungen mittels eines Corticosteroids zusammen mit Exosomen. Diese kann in Gegenwart oder Abwesenheit eines Cytokin-Antagonisten und/oder eines Wachstumsfaktors erfolgen. Eine Gegenwart des Cytokin-Antagonisten und/oder des Wachstumsfaktors ist bevorzugt.

Diese unterschiedlichen Wirkstoffe können dabei gleichzeitig - in derselben Formulierung oder in unterschiedlichen Formulierungen - oder auch sequenziell verabreicht werden. Die erfindungsgemäßen pharmazeutischen Zusammensetzungen, die nur einen der beiden Wirkstoffe Exosomen und Corticosteroid enthalten, ebenso wie der erfindungsgemäße Kit, können daher einerseits für eine simultane Verabreichung und andererseits für eine sequenzielle Verabreichung der Exosomen und des Corticosteroids bestimmt sein. Eine gleichzeitige Verabreichung ist allerdings bevorzugt, insbesondere in nur einer Formulierung. So können zum Beispiel die beiden pharmazeutischen Zusammensetzungen des erfindungsgemäßen Kits vor einer Verabreichung an den Patienten in geeignetem Verhältnis gemischt werden und dann als eine Formulierung verabreicht werden. Bei einer sequenziellen Verabreichung der Exosomen und des Corticosteroids werden die verschiedenen Wirkstoffe vorzugsweise innerhalb eines Zeitraums von einer Woche, vorzugsweise innerhalb von 5 Tagen, 3 Tagen, einem Tag, oder innerhalb von 12 Stunden verabreicht.

In den erfindungsgemäßen pharmazeutischen Zusammensetzungen, die Exosomen und/oder ein Corticosteroid enthalten, kann ein Cytokin-Antagonist und/oder ein Wachstumsfaktor anwesend oder abwesend sein. Eine Gegenwart des Cytokin-Antagonisten und/oder des Wachstumsfaktors ist bevorzugt. Es tritt nämlich ein deutlicher vorteilhafter Effekt auf die Wirksamkeit, insbesondere die Langzeit-Wirksamkeit, von Corticosteroiden bei Erkrankungen der Gelenke und der Wirbelsäule auf, wenn zusätzlich ein Cytokin-Antagonist wie Orthokin (natürliches IL-1 Ra) und Anakinra (rekombinantes IL-1Ra) verabreicht wird, insbesondere bei der lokalen Verabreichung der Therapeutika direkt in das zu behandelnde Gelenk. Eine ähnliche, überraschend gute Wirksamkeit der Kombination dieser Wirkstoffe wurde auch für Autoimmunkrankheiten beobachtet, wobei vor allem die entzündungshemmende Wirkung eine Rolle spielt. Die Wirksamkeit einer Kombinationstherapie mit Corticosteroiden und Exosomen bei Erkrankungen der Gelenke und der Wirbelsäule wie Arthrose, Arthritis und degenerativer Wirbelsäulenerkrankung wird durch die zusätzliche Verabreichung eines Cytokin-Antagonisten ebenfalls erheblich verbessert. Bei einer Behandlung mit dem rekombinanten IL-1Ra Anakinra ergibt sich überraschenderweise erst durch die Kombination mit einem Corticosteroid eine Wirksamkeit. Bei dem natürlichen IL-1Ra Orthokin ist eine deutliche Verbesserung der Wirksamkeit gerade bei entzündlichen Erkrankungen zu beobachten. Eine mögliche Erklärung für diesen Umstand ist, dass die Cytokin-Antagonisten eine anabole Wirkung haben und die in den betroffenen Gelenken bekannte schädliche katabole Wirkung des Corticosteroids aufheben oder gar umkehren können. Daher können die Corticosteroide bei der Behandlung von zum Beispiel Arthrose neben den Cytokin-Antagonisten alternativ oder zusätzlich auch mit anabolen Wachstumsfaktoren kombiniert werden, um eine ähnliche Wirkung zu erzielen. Allgemein kann der Cytokin-Antagonist auch durch einen Wachstumsfaktor ersetzt werden oder mit einem Wachstumsfaktor kombiniert werden.

Diejenige erfindungsgemäße pharmazeutische Zusammensetzung (gemäß dem vierten Aspekt der vorliegenden Erfindung), die einen Cytokin-Antagonisten und/oder einen Wachstumsfaktor zur Verwendung in einer Kombinationstherapie zusammen mit einem Corticosteroid und Exosomen enthält, kann einerseits für eine simultane Verabreichung und andererseits für eine sequenzielle Verabreichung des Cytokin-Antagonisten und/oder Wachstumsfaktors, der Exosomen und des Corticosteroids bestimmt sein. Eine gleichzeitige Verabreichung ist bevorzugt, insbesondere in nur einer Formulierung. So kann beispielsweise der Cytokin-Antagonist und/oder Wachstumsfaktor vor einer Verabreichung an den Patienten in geeignetem Verhältnis mit einem Corticosteroid und Exosomen gemischt werden und dann als eine Formulierung verabreicht werden. Alternativ kann (können) bei gleichzeitiger Verabreichung auch der Cytokin-Antagonist und/oder Wachstumsfaktor, die Exosomen oder aber das Corticosteroid gesondert verabreicht werden und die anderen Bestandteile als eine Formulierung gemischt werden. Es können allerdings Cytokin-Antagonist und/oder Wachstumsfaktor, Exosomen und Corticosteroid auch in beliebiger Reihenfolge sequenziell verabreicht werden, oder einer dieser Wirkstoffe kann zu einem anderen Zeitpunkt als die beiden anderen verabreicht werden. Bei einer sequenziellen Verabreichung werden die verschiedenen Wirkstoffe vorzugsweise innerhalb eines Zeitraums von einer Woche, vorzugsweise innerhalb von 5 Tagen, 3 Tagen, einem Tag, oder innerhalb von 12 Stunden verabreicht.

Bei den anderen erfindungsgemäßen pharmazeutischen Zusammensetzungen ebenso wie beim erfindungsgemäßen Kit und der erfindungsgemäßen Verwendung (erster, zweiter, dritter, fünfter, sechster und siebter Aspekt der vorliegenden Erfindung) ist die Gegenwart oder die Abwesenheit eines Cytokin-Antagonisten in der pharmazeutischen Zusammensetzung möglich. Die Gegenwart eines Cytokin-Antagonisten ist bevorzugt. Außerdem ist die erfindungsgemäße Kombinationstherapie vorzugsweise eine Kombinationstherapie zusammen mit einem Cytokin-Antagonisten, insbesondere in den Fällen, in denen in der pharmazeutischen Zusammensetzung selbst ein Cytokin-Antagonist abwesend ist (siehe oben).

Der erfindungsgemäß verwendete Cytokin-Antagonist kann ein jeglicher Stoff oder ein jegliches Stoffgemisch sein, das im Körper des Patienten mindestens eine, vorzugsweise im wesentlichen alle der biologischen Aktivitäten von einem oder mehreren Cytokinen verringert oder hemmt. Die antagonistische Wirkung kann dabei direkt durch den Antagonisten oder indirekt geschehen, zum Beispiel durch Aktivierung oder Hemmung weiterer Signalwege, die ihrerseits auf die biologische Aktivität des Cytokins einwirken. Vorzugsweise wird die biologische Aktivität des Cytokins durch Blockierung seiner Interaktion mit einem oder mehreren der Rezeptoren, an die es binden kann, gehemmt. Dies kann zum Beispiel durch kompetitive Bindung des Antagonisten an den/die entsprechenden Rezeptoren oder durch Bindung des Antagonisten an das Cytokin selbst erreicht werden. Vorzugsweise hemmt der Cytokin-Antagonist die Wirkung des Cytokins IL-1.

Der Cytokin-Antagonist kann beispielsweise ein Protein, ein Peptid, eine Nukleinsäure, ein Lipid oder eine organische Verbindung sein. Auch kann der Cytokin-Antagonist aus einem Gemisch von zwei oder mehr Cytokin-Antagonisten wie sie hierin beschrieben sind bestehen. Insbesondere kann der Cytokin-Antagonist ein natürlich vorkommendes Peptid oder Protein oder auch ein rekombinant hergestelltes Peptid oder Protein sein. Außerdem kann der Cytokin-Antagonist ein Antikörper oder ein Antigen-bindendes Fragment eines Antikörpers sein oder enthalten, insbesondere ein Antikörper oder Antikörperfragment, das das betreffende Cytokin oder einen Cytokin-Rezeptor binden kann. Beispiele für geeignete Cytokin-Antagonisten sind Interleukin-Antagonisten, insbesondere IL-1-Antagonisten wie IL-1Ra, Tumornekrosefaktor (TNF)-Antagonisten, insbesondere ein TNF-α-Antagonist wie ein Anti-TNF-α-Antikörper, Interferon-Antagonisten, und Chemokin-Antagonisten. Besonders bevorzugt ist natürlich vorkommendes oder rekombinantes IL-1 Ra-Protein, vorzugsweise humanes IL-1 Ra. IL-1Ra umfasst vorzugsweise oder besteht vorzugsweise aus der Aminosäuresequenz einer Isoform oder eines Homologs des humanen IL-1Ra gemäß SEQ ID NOs: 1, 2, 3, 4 oder 5, einer Isoform des equinen IL-1 Ra gemäß SEQ ID NOs: 6 oder 7, oder einer Isoform des caninen IL-1Ra gemäß SEQ ID NO: 8.

Außerdem können erfindungsgemäß Fragmente oder Derivate von IL-1Ra als Cytokin-Antagonisten verwendet werden, solange diese die gewünschte Funktion, das heißt das Verringern oder Hemmen einer oder mehrerer biologischer Funktionen von IL-1, ausüben können. Fragmente von IL-1 Ra umfassen vorzugsweise mindestens 20, mehr bevorzugt mindestens 40, 60, 80 oder mindestens 100 Aminosäuren einer natürlichen IL-1Ra-Sequenz. Vorzugsweise sind die Fragmente natürlicherweise vorkommende sekretierte Fragmente von IL-1Ra. In einer Ausführungsform umfasst das IL-1Ra die Aminosäuren 26 bis 177 des humanen IL-1Ra, vorzugsweise Aminosäuren 26 bis 177 der Sequenz gemäß SEQ ID NO: 1. Derivate von IL-1Ra sind vorzugsweise homolog zu natürlichem IL-1Ra und haben bevorzugt eine Homologie oder Identität zu natürlichem IL-1Ra von mindestens 60%, mehr bevorzugt mindestens 70%, 75%, 80%, 85%, 90%, 95% und am meisten bevorzugt mindestens 98% über einen Bereich von mindestens 20 zusammenhängenden Aminosäuren, bevorzugt mindestens 40, 60, 80 oder mindestens 100 zusammenhängenden Aminosäuren, am meisten bevorzugt über die gesamte Länge von IL-1Ra. Besonders bevorzugt sind das aus natürlichen biologischen Proben wie Blut isolierbare IL-1Ra, auch Orthokin genannt, sowie das IL-1 Ra Fragment mit den Aminosäuren 26 bis 177 des humanen IL-1Ra, auch Anakinra genannt. Die Gewinnung von Orthokin ist unter anderem in den Patentanmeldungen WO 00/46249 A1 und WO 03/080122 A1 beschrieben. Anakinra sowie weitere IL-1-Antagonisten, die in dieser Erfindung verwendet werden können, sind unter anderem in der Patentanmeldung EP 0 343 684 A1 beschrieben.

Bei der Gewinnung von IL-1Ra aus natürlichen biologischen Proben wie Blut, wie z.B. bei Orthokin, enthält die erhaltene IL-1Ra-Lösung vorzugsweise auch Wachstumsfaktoren. Daher kann ein Cytokin-Antagonist erfindungsgemäß auch in Kombination mit einem oder mehreren Wachstumsfaktoren vorliegen oder durch einen oder mehrere Wachstumsfaktoren ersetzt werden. Der Wachstumsfaktor hat erfindungsgemäß vorzugsweise eine anabole Wirkung. Beispiele für geeignete Wachstumsfaktoren sind TGF-β, IGF, BMP, HGF und VEGF. Auch umfasst sind Analoge, Derivate und Fragmente dieser Wachstumsfaktoren, solange diese die gewünschte Wirkung, d.h. insbesondere ihre Wirkung als Wachstumsfaktor aufweisen.

Das erfindungsgemäß verwendete Corticosteroid kann ein jegliches natürlich vorkommendes sowie synthetisch hergestelltes Corticosteroid sein. Insbesondere kann es ein Glucocorticoid, ein Mineralocorticoid oder ein Androgen sein, wobei Glucocorticoide bevorzugt verwendet werden. Ebenso kann auch ein Gemisch aus zwei oder mehreren Corticosteroiden wie hierin beschrieben verwendet werden. Beispiele für Glucocorticoide sind Cortison, Hydrocortison, Prednison, Prednisolon, Cloprednol, Deflazacort, Fluocortin, Triamcinolon, Dexamethason, Methylprednisolon, Fluprednisolon, Clocortolon, Clobetason, Alclomethason, Flumethason, Fluopredniden, Fluorandrenolon, Betamethason, Beclomethason, Fluocortolon, Mometason, Fluticason, Halomethason, Fluocinolon, Diflorason, Desoximethason, Fluocinonid, Amcinonid, Halcinonid, Diflucortolon, Clobetasol und Paramethason. Beispiele für Mineralocorticoide sind Aldosteron, Desoxycorticosteron und Fludrocortison, und Beispiele für Androgene sind Dehydroepiandrosteron (DHEA) und Estrogene. Das Corticosteroid kann als freie Verbindung oder in Form eines Salzes, Esters oder Prodrugs eingesetzt werden. In bevorzugten Ausführungsformen ist das verwendete Corticosteroid Triamcinolon, Cortison, Hydrocortison, Prednisolon oder Prednison.

Die Exosomen in den erfindungsgemäßen Exosomen-enthaltenden pharmazeutischen Zusammensetzungen bzw. in dem erfindungsgemäßen Kit sind herstellbar durch ein Verfahren umfassend die Schritte: Bereitstellen einer Exosomen enthaltenden Blutprobe und vorzugsweise Aufkonzentrieren der Exosomen. Das Aufkonzentrieren erfolgt vorzugsweise durch einen Zentrifugationsschritt mit mindestens 100 000 g, da solch hohe Zentrifugalbeschleunigungen besonders geeignet sind, um Exosomen zu konzentrieren. Dieser Schritt wird vorzugsweise mindestens 30 min, insbesondere mindestens 60 min durchgeführt, da das Aufkonzentrieren dann besonders effektiv ist.

Der Schritt des Bereitstellens einer Exosomen enthaltenden Blutprobe umfasst vorzugsweise die Schritte: Bereitstellen einer einem Patienten entnommenen Blutprobe, optional Hinzufügen eines die Exosomenbildung fördernden Zusatzstoffes, und Inkubieren der Blutprobe in einem für die Exosomenherstellung geeigneten Gefäß. Das Inkubieren führt zur Bildung einer konditionierten Blutzusammensetzung. Für die Exosomenherstellung geeignete Gefäße sind beispielsweise Spritzen, Röhrchen wie etwa Vakuumröhrchen, Mikrotiterplatten und Transfusionsbeutel. Die Oberfläche zur Kontaktierung der Blutprobe umfasst bei für die Exosomenherstellung geeigneten Gefäßen vorzugsweise Glas, Kunststoff (z.B. Polystyrol, Polyvinylchlorid, Polyethylen oder Polypropylen), Korund oder Quarz und besteht bevorzugt aus einem dieser Materialien. Vorzugsweise werden zur Exosomenherstellung in diese Gefäße oberflächenvergrößernde Zusätze aus Glas, Kunststoff, Korund oder Quarz, wie etwa Kugeln, Gele, Wolle, Mehl, Granulate oder Partikel zugefügt. Der die Exosomenbildung fördernde Zusatzstoff ist vorzugsweise IL-1Ra. Der die Exosomenbildung fördernde Zusatzstoff wird bevorzugt in einer Menge von 1 bis 20 µg pro ml Vollblut eingesetzt.

Bei den erfindungsgemäßen pharmazeutischen Zusammensetzungen zur Verwendung in einer Kombinationstherapie zusammen mit Exosomen gilt, dass die Exosomen aus einer Blutprobe gewonnen wurden. Die Exosomen sind vorzugsweise bezüglich des zu behandelnden Patienten autolog oder allogen. Hinsichtlich der Gewinnung der Exosomen wird auf die soeben gemachten Ausführungen zum Herstellen von Exosomen verwiesen. Vorzugsweise wird der oben beschriebene Zentrifugationsschritt mit mindestens 100 000 g (vorzugsweise mindestens 30 min, insbesondere mindestens 60 min) bei der Behandlung von Krankheiten durchgeführt, bei denen eine erhöhte Konzentration von Exosomen sinnvoll ist, bevorzugt gegebenenfalls bei der Behandlung von rheumatoider Arthritis. Besonders bevorzugt wird ein solcher Zentrifugationsschritt ganz allgemein durchgeführt für den Fall, dass die Kombinationstherapie Exosomen involviert, die aus einer Blutprobe eines Patienten gewonnen werden.

Die oben gemachten näheren Ausführungen zum Herstellen von Exosomen gelten entsprechend für das erfindungsgemäße Verfahren zur Herstellung einer ein Corticosteroid und Exosomen enthaltenden pharmazeutischen Zusammensetzung umfassend die Schritte: Bereitstellen einer Exosomen enthaltenden Blutprobe, vorzugsweise Aufkonzentrieren der Exosomen und Vermischen mit einem Corticosteroid. Das Vermischen kann in einer beliebigen dem Fachmann geläufigen Weise erfolgen.

In (gegebenenfalls bevorzugten) Ausführungsformen sind die erfindungsgemäßen pharmazeutischen Zusammensetzungen bzw. der erfindungsgemäße Kit für eine Verwendung bei der Behandlung von Gelenkerkrankungen wie Arthrose, Arthritis, Gelenkentzündung und entzündlichem Knorpelverlust, degenerativen Wirbelsäulenerkrankungen, Gelenkschmerzen und auch Autoimmunerkrankungen bestimmt. Die zu behandelnde Arthrose kann durch Überbelastung entstanden sein, angeborene oder traumatische Ursachen haben oder die Folge einer anderen Erkrankung wie einer Entzündung sein. Die zu behandelnde Arthrose ist vorzugsweise eine aktivierte Arthrose oder eine entzündliche Arthrose. Die erfindungsgemäßen pharmazeutischen Zusammensetzungen können für die Behandlung von Arthrosen und Arthritis an jedem beliebigen Gelenk eingesetzt werden, wie zum Beispiel Kniegelenk, Hüftgelenk, Sprunggelenk, Schultergelenk, Wirbelgelenke, Fingergelenke, Ellenbogengelenk, Zehengelenke, Kiefergelenk und Handgelenk. Die zu behandelnde Arthritis kann eine infektionsbedingte Arthritis wie bakterielle Arthritis oder eine nicht-infektionsbedingte Arthritis wie gegebenenfalls rheumatoide Arthritis, oder aber psoriatische Arthritis oder Gicht-Arthritis sein. Alternativ können die erfindungsgemäße pharmazeutische Zusammensetzung und/oder der erfindungsgemäße Kit auch zur Verwendung bei der Behandlung einer von einer oder mehreren der genannten Krankheiten (etwa rheumatoider Arthritis) verschiedenen Krankheit bestimmt sein. Die zu behandelnde degenerative Wirbelsäulenerkrankung kann beispielsweise ein Bandscheibenvorfall sein. Autoimmunerkrankungen umfassen unter anderem Autoimmunerkrankungen der Gelenke wie zum Beispiel Morbus Bechterew, gegebenenfalls rheumatoide Arthritis und systemischer Lupus erythematodes, sowie andere Autoimmunerkrankungen wie insbesondere Neurodermitis und Alopecia areata (kreisrunder Haarausfall).

Die erfindungsgemäßen pharmazeutischen Zusammensetzungen bzw. der erfindungsgemäße Kit sind vorzugsweise zur lokalen Verabreichung geeignet. Sie sind bevorzugt zur lokalen Verabreichung bestimmt. Daher sind sie in bevorzugten Ausführungsformen zur Injektion, insbesondere zur Injektion in die zu behandelnde Körperregion, insbesondere in das betroffene Gelenk, an die betroffene Nervenwurzel oder in die betroffene Bandscheibe, oder in die lokale Umgebung hiervon bestimmt. Die pharmazeutische Zusammensetzung ist somit insbesondere zur intraartikulären und/oder periradikulären Injektion bestimmt. Alternativ können die erfindungsgemäßen pharmazeutischen Zusammensetzungen für eine topische Verabreichung formuliert sein, insbesondere als Creme oder Gel, oder für eine systemische Verabreichung, insbesondere eine orale Verabreichung in Form von Tabletten, Kapseln oder Pastillen. Die Verabreichungsform hängt unter anderem von der zu behandelnden Krankheit ab. Bei einer lokalen Arthrose oder einer degenerativen Wirbelsäulenerkrankung ist daher eine lokale Verabreichung der erfindungsgemäßen pharmazeutischen Zusammensetzungen bevorzugt. In bevorzugten Ausführungsformen sind die erfindungsgemäßen pharmazeutischen Zusammensetzungen bzw. der erfindungsgemäße Kit ausschließlich für eine von einer systemischen Verabreichung verschiedene Verabreichung bestimmt oder geeignet.

Die erfindungsgemäßen pharmazeutischen Zusammensetzungen sind für die verschiedenen Verabreichungswege jeweils geeignet in dem Fachmann bekannter Weise formuliert. So liegt eine für eine Injektion geeignete pharmazeutische Zusammensetzung vorzugsweise als Lösung oder Dispersion oder auch in trockener Form z.B. als Pulver oder Lyophilisat vor, welches vor der Injektion in einem geeigneten Lösungsmittel wie Wasser gelöst werden muss. Die erfindungsgemäßen pharmazeutischen Zusammensetzungen enthalten die Exosomen und/oder das Corticosteroid in therapeutisch wirksamen Mengen. Das Corticosteroid liegt vorzugsweise in einer Konzentration von 1 bis 80 mg/Dosis, mehr bevorzugt von 5 bis 40 mg/Dosis in den Corticosteroid-enthaltenden pharmazeutischen Zusammensetzungen vor. Ein gegebenenfalls vorhandener Cytokin-Antagonist ist vorzugsweise in einer Konzentration von 0,5 bis 150 mg/Dosis in den Cytokin-Antagonist-enthaltenden pharmazeutischen Zusammensetzungen vorhanden, kann aber auch in erheblich geringerer Konzentration wie etwa 1 ng/Dosis oder mehr, zum Beispiel zwischen 1 und 1000 ng/Dosis vorliegen. Diese geringen Dosiskonzentrationen können insbesondere bei einer Kombination mit Wachstumsfaktoren und/oder bei natürlichen IL-1Ra-Präparaten wie beispielsweise bei Zusammensetzungen mit Orthokin verwendet werden. Die höheren Dosiskonzentrationen sind beispielsweise bei rekombinant hergestellten Cytokin-Antagonisten wie Anakinra bevorzugt. Außerdem können die erfindungsgemäßen pharmazeutischen Zusammensetzungen ferner einen oder mehrere Trägerstoffe und/oder ein oder mehrere Exzipienten enthalten.

Die pharmazeutischen Zusammensetzungen der Erfindung können auch für eine Behandlung von Patienten bestimmt sein, die bereits einer anderen Behandlung der relevanten Krankheit, d.h. zum Beispiel einer Arthrose, Arthritis, und/oder degenerativen Wirbelsäulenerkrankung, unterzogen wurden, insbesondere wenn diese andere Behandlung nicht erfolgreich war oder die Krankheitssymptome trotz anfänglich erfolgreicher Behandlung zumindest teilweise zurückgekehrt sind. In bevorzugten Ausführungsformen ist diese andere Behandlung eine Therapie mit Exosomen, aber ohne ein Corticosteroid, oder eine Therapie mit einem Corticosteroid, insbesondere einem Glucocorticoid wie vorstehend beschrieben, aber ohne Exosomen.

Patienten im Sinne der Erfindung können Menschen sowie Tiere sein, die unter einer der hierin beschriebenen Krankheiten leiden. Somit können die erfindungsgemäßen pharmazeutischen Zusammensetzungen geeignet sein für die Behandlung eines Menschen und/oder eines Tieres wie zum Beispiel eines Hundes, einer Katze, eines Pferdes, eines Rinds, eines Schweins, einer Ziege, eines Kamels oder dergleichen.

### Kurzbeschreibung der Figuren

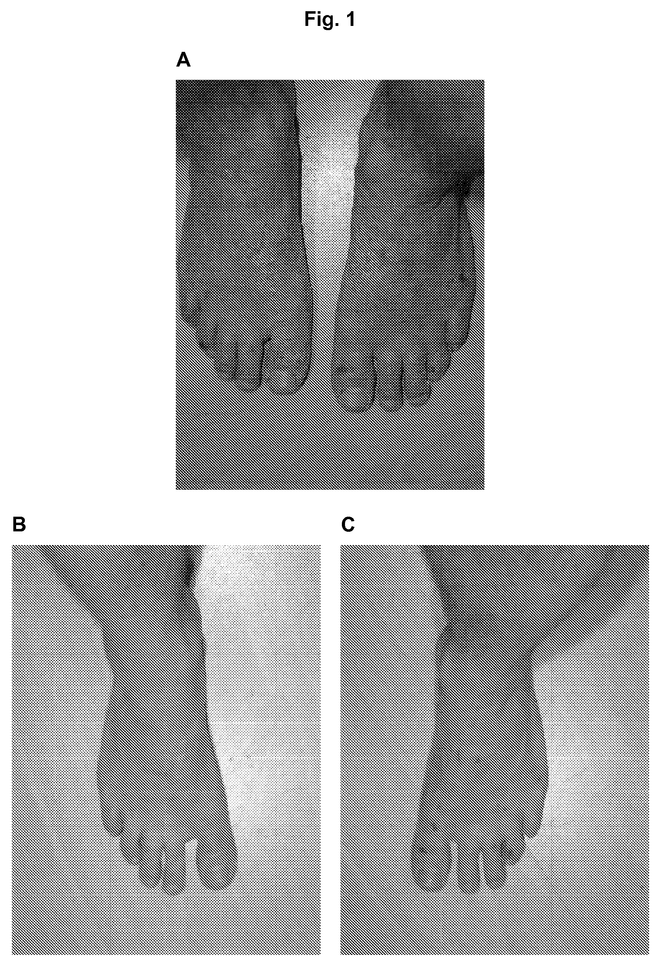

Fig. 1A bis Fig. 1C zeigen Aufnahmen der Füße eines Neurodermitis-Patienten, dessen Fall unten als Fall IX geschildert ist (Beispiel 4).
Fig. 1A gibt die dermatologischen Veränderungen an beiden Füßen unmittelbar vor der Behandlung mit Exosomen und Triamcinolon wieder.
Fig. 1B zeigt den Zustand eine Woche nach Behandlung für den rechten Fuß.
Fig. 1C zeigt den Zustand eine Woche nach Behandlung für den linken Fuß.

### Beispiele

Im Folgenden sind verschiedene Fallstudien von Patienten mit fortgeschrittener Arthrose beschrieben. Diese wurden mit einer Kombinationstherapie mit einem Cytokin-Antagonisten (z.B. rekombinantes IL-1Ra rekombinant oder aus autologen Blutproben gewonnenes IL-1 Ra) und einem Corticosteroid behandelt.

**Abkürzungen:**

| | |
|---|---|
| re | rechts |
| li | links |
| bds | beidseitig |
| IRO | Innenrotation |
| ARO | Außenrotation |
| VAS | visuelle Analogskala für Schmerzempfindung (0 bis 10) |
| WOMAC | Patientenfragebogen zu Arthrose |
| CRP | c-reaktives Protein, ein im Blut nachweisbarer Entzündungsmarker |
| OSG | Oberschenkelgelenk |

### Therapie mit Orthokin und Cortison bei Arthrose:

Patientenzahl: N = 129
durchschnittlicher Nachuntersuchungszeitraum: 3 Monate
durchschnittliche Schmerzreduktion: 71% (d.h. Reduktion von 100% Schmerz vor Behandlung auf 29% nach Behandlung)
   auffallend schneller Wirkungseintritt

### 1. Lokale Verabreichung von Anakinra und Cortison und Exosomen

Fall I: T., 56 Jahre, weiblich
*Diagnose:* Klinisch radiologisch besteht mediale und retropatellare Gonarthrose li, Grad IV. Auswärtig wurde bereits ein totaler Knieersatz links geplant;
*Therapie:* 3 Injektionen von Exosomen kombiniert mit Anakinra und 10 mg Triamcinolon in das linke Knie (2x wöchentlich) unter Vermeidung einer Knieoperation.
*Ergebnis:* Zum Zeitpunkt der 3. Injektion 100% Schmerzbesserung, deutliche Funktionsverbesserung. Operationstermin wurde abgesagt, Patientin war auch 5 Monate nach Therapieende noch schmerzfrei.

### 2. Lokale Verabreichung von Anakinra und Cortison und Orthokin

Fall II: L., 57 Jahre, männlich
*Diagnose:* Starke Schulterschmerzen li seit 6 Monaten (VAS 8); seitdem deutlich gestörte Nachtruhe. Patient konnte in den letzten 6 Monaten kaum schlafen, deswegen auch Störung des allgemeinen Wohlbefindens. Zahlreiche Injektionen mit Cortison in die linke Schulter waren ohne Erfolg. Ein Operationstermin für die linke Schulter war vereinbart. Hier sollte ein Versuch unternommen werden, die Operation zu vermeiden. Radiologisch und klinisch Zeichen der partiellen Rotatorenmanschettenruptur und der subakromialen Enge mit kompletter Schultersteife links; Missempfindungen linker Arm mit Schwäche der Kraft von Hand und Unterarm links, Grad 4.
*Therapie:* Die Injektionen wurden in die linke Schulter dorsal und lateral appliziert. 2 ml Orthokin wurden mit 10 mg Anakinra und 10 mg Triamcinolon über eine Spritze in die linke Schulter appliziert. Die Therapie wurde an 4 aufeinander folgenden Tagen durchgeführt.
*Ergebnis:* Bereits am 2. Behandlungstag gab der Patient eine extreme Schmerzbesserung mit einer Schmerzreduktion um 90% an. VAS war von 8 auf 1 gefallen, die Schulter war frei und normal beweglich. Der Patient konnte erstmalig seit 6 Monaten durchschlafen. Der Patient war dadurch in seinem allgemeinen Wohlbefinden deutlich gebessert. Fortsetzung der Therapie bis Behandlungstag 4. Weiterhin zeigte sich eine unverändert deutliche Besserung wie an Behandlungstag 2, bei der Nachkontrolle 6 Monate nach Behandlung gab es einen unverändert positiven Befund. Die Operation wurde abgesagt, Beweglichkeit war frei, der Patient kann wieder problemlos Koffer und Bücher über Schulterhöhe hinaus heben.

Fall III: F., 45 Jahre, weiblich
*Diagnose:* Komplette Schultersteife rechts seit ca. 8 Monaten. Alle bisherigen Therapien waren ohne Erfolg, eine OP war geplant. Patientin wollte Versuch der erneuten konservativen Behandlung. Der Nachtschlaf war seit einigen Wochen nicht mehr möglich. Links beginnende Schulterschmerzen, Hauptbefund aber rechte Schulter, dort VAS 9 mit schweren akuten Attacken bis auf 10, dadurch insgesamt reduzierter Allgemeinzustand.
*Therapie:* Behandlung der rechten Schulter aus einer Kombination von 2 ml Orthokin getrennt appliziert zusammen mit einer anderen Spritze aus einer Kombination von 150 mg Anakinra und 5 mg Triamcinolon an 6 aufeinander folgenden Tagen.
*Ergebnis:* Ab dem 5. Tag 85% Schmerzbesserung. Nächtliches Durchschlafen war seit 2. Behandlung möglich, dadurch erheblich gebesserter Allgemeinzustand. VAS bei Behandlungsende bei 1. Nachkontrolle 8 Monate nach Behandlung zeigte weiter ein sehr gutes unverändertes Ergebnis; OP wurde abgesagt.

### 3. Exosomen inkubiert mit IL-1Ra und Triamcinolon/Prednisolon

### Fall IV: S., 25 Jahre, männlich

*Diagnose:* Schwere juvenile rheumatoide Arthritis seit ca. 15 Jahren.
   Behandlung mit 25 mg Enbrel 2 x pro Woche, 10 mg Methotrexat, 5 mg Decortin und Naproxen 2x1 pro Tag. Massive Synovitis und Schmerzen beide OSG und beide Schultern. Abduktion beider Schultern vor Behandlung 60 Grad. Labor CRP-Wert: 5,35 (bis 0,5 mg ist normal); Leukozytose.
*Therapie:* Es wurde Blut abgenommen zur Herstellung von Exosomen in einer 6 ml Spritze (Orthokinspritze). Danach 24 h Inkubation bei 37 Grad, wobei bei Füllung der Spritze mit Blut vorher 1 mg Anankinra (IL-1Ra) und 2 mg Prednisolon in die Spritze appliziert wurden. Nach verschiedenen Schritten der Zentrifugation (bis 100 000 g) wurde das Gemisch dann der Patientin in die OSGe und die Schultern appliziert.
*Ergebnis:* Nach 3 Tagen beginnende deutliche Abschwellung der Gelenke. Klinische und laborchemische Kontrolle nach 9 Tagen: 80% Schmerzbesserung, OSG normal, keine Schwellung. CRP-Wert jetzt auf 1,93. Verbesserung auch in anderen betroffenen Gelenken, die nicht lokal injiziert wurden. Allgemeine Lebensqualität erheblich verbessert. Bei der Kontrolle nach 3 Monaten zeigt sich eine unverändert stabile Situation. VAS vor Behandlung 9, seit der ersten Woche nach Injektion VAS 3. Patient sehr zufrieden, kann Arbeit wieder fortsetzen.

### 4. Injektion mit Exosomen und zusätzlichem Triamcinolon bei Injektion

### Fall V: M., 64 Jahre, weiblich

Schwere therapieresistente RA trotz Basistherapie mit Prednisolon 15 mg p.d., Lantarel 20 mg p.W., Humira alle 2 Wochen. Radiosynovioorthese in die Handgelenke zeigte nur einen minimalen Effekt, intraartikuläre Injektionen in Dosierungen zwischen 10 mg bis 40 mg Triamcinolon zeigten nur sehr schwachen (20% Schmerzbesserung nach einer Woche) Effekt sowohl auf den Schmerz als auch auf die Entzündungsparameter. Bei der Vorstellung zur Exosomentherapie CRP 120 mg/l trotz oben beschriebener Basistherapie, sehr starke Schmerzen in den Händen und beiden Schultern. Durchführung einer Injektion von Exosomen (nach Inkubation mit IL-1Ra) und Zumischung von 20 mg Gesamtmenge Triamcinolon in MCP2-5 bds und bd. Schultern. Daraufhin klinische starke Besserung (80% Schmerzreduktion nach 1 Woche) beginnend nach 2 Tagen, die dauerhaft über 3 Monate anhält. CRP Kontrolle nach 3 Monaten CRP 42,6 mg/l, Basistherapie unverändert, so dass die Wirkung auf die Kombination Exosomen mit Triamcinolon zu beziehen ist. Höhere Dosierungen von alleinigem Triamcinolon wie oben beschrieben zeigten keine vergleichbaren Wirkungen.

### Fall VI: M., 25 Jahre, männlich

Bekannte Psoriasisarthritis; Basistherapie 5 mg Prednisolon und 10 mg MTX; Hauptproblem unter Basistherapie noch deutliche Schwellungen linkes Knie mit Synovitis und Schwellung von 2 cm verglichen mit der Gegenseite. Zusätzlich Schwellungen im Bereich der Handgelenke trotz durchgeführter Basistherapie. Intraartikuläre Cortisoninjektionen in die Knie und Handgelenke in Dosierungen zwischen 20-40 mg zeigten nur einen sehr schwachen Effekt über einige Tage mit Schmerzreduktionen zwischen 10-30%. Vorstellung wegen Exosomentherapie. CRP unmittelbar vor Injektion 5,6 mg/l. Nach Fertigstellung der Exosomen Applikation von Exosomen (hergestellt nach IL-1Ra Inkubation wie beschrieben) in das linke Knie (Exosomen + Triamcinolon 10 mg) und in die MCP 2+3 bds (Exosomen mit je 2 mg Triamcinolon je Gelenk). Komplikationsloser Verlauf, nach einigen Tagen deutliche Schmerzlinderung von 90%; CRP nach 3 Monaten 3,0 mg/l; Knie nach 1 Woche komplett abgeschwollen, kein Seitenunterschied: 5 mg Prednisolon konnte im Rahmen der Basistherapie komplett abgesetzt werden, da der Effekt der intraartikulären Injektion aus Exosomen und Triamcinolon andauernd war.

### Fall VII: R., 32 Jahre, männlich

Komplette Alopezie seit Jahren bekannt, sämtliche Therapieversuche inkl. systemsicher und lokaler Cortisonapplikation in hohen systemischen und lokalen Dosen (10-80 mg Prednisolon) ohne Wirkung: Vorstellung zur Behandlung mit Exosomen. Herstellung der Exosomen in oben beschriebener Technik, einmalige intramuskuläre Injektion der Exosomen zusammen mit 10 mg Triamcinolon. Verlaufskontrollen unauffällig, nach 3 Monaten Nachweis von Haarwuchs in etwa der Hälfte der ursprünglich vor Erkrankung behaarten Fläche.

### Fall VIII: H., 47 Jahre, männlich:

Gonarthrose re mit Knorpeldefekten arthroskopisch verifiziert Grad 2-4 nach Outerbridge. Der Patient wünscht Herauszögern einer OP und eines Gelenkersatzes. Anamnestisch Injektion von Triamcinolon in Dosierungen von 10-40 mg intraartikulär keinerlei Wirkung. Injektion von Exosomen 1 ml hergstellt in der Technik wie beschrieben nach Inkubation mit IL-1Ra. Intraartikuläre Injektion einmalig von 2 ml Exosomen, nach 4 Wochen Patient mit klinischem Effekt in Bezug auf Schmerz und Funktion unzufrieden. Daraufhin Entscheidung einer intraartikulären Injektion in das rechte Knie kombiniert mit 10 mg Triamcinolon. Komplikationsloser Verlauf. Nach 1 Woche 95%-ige Schmerzbesserung, Funktion wieder komplett hergestellt, Patient kann seit Jahren erstmalig wieder an einem Tennismatch teilnehmen.

### Fall IX: J., 27 Jahre, männlich:

Schwere dermatologisch gesicherte Neurodermitis mit starken Veränderungen v.a. Im Bereich der Hände und Füße. Sämtliche bekannten dermatologischen Therapien zeigten keine nachhaltige Besserung, insbesondere war nur ein kurzzeitiger Effekt von 1 Woche nach lokaler Applikation von Corticosteroiden und systemischer Applikation von Prednisolon und Triamcinolon intramuskulär in Dosierungen von 80 mg zu beobachten. Herstellung von Exosomen in beschriebener Technik, Applikation i.m.; Nach 3 Wochen kein zufriedenstellender Effekt auf die Hautveränderungen und die Krankheit. Daraufhin Versuch der Applikation i.m. von 2 ml Exosomen in Verbindung mit 20 mg Triamcinolon. Innerhalb von 2 Wochen deutliche Verbesserung, die auch über 6 Monate unverändert anhält. Danach leichte Verschlechterung, aber weiterhin Besserung verglichen zum Vorbefund. Fig. 1A beschreibt die dermatologischen Veränderungen an beiden Füßen unmittelbar vor Behandlung, Fig. 1B und Fig. 1C eine Woche nach Behandlung für den rechten bzw. linken Fuß.

### Fall X, B., 69 J., weiblich

Schwere Bechterewarthritis mit Iridocyclitis linkes Auge. Patientin bekommt als Basistherapie Prednisolon und MTX in wechselnden Dosierungen. CRP 20,3 mg/l; linkes OSG deutlich geschwollen mit + 2 cm gegenüber der anderen Seite; linkes MCP und Endgelenk 1. Finger links deutlich geschwollen und schmerzhaft, rechte Schulter geschwollen, schmerzhaft, Abduktion um 30 Grad gegenüber normal vermindert. Daraufhin Injektion von Exosomen in bekannter Technik in die betroffenen Gelenke ohne Schmerzminderung und CRP Verminderung, daraufhin Injektion von Triamcinolon in einer Gesamtdosis von 40 mg in alle betroffenen Gelenke, leichte Besserung von 20% über 1 Woche. Nach 4 Wochen Injektion von Exosomen und Triamcinolon Gesamtdosis 20 mg, daraufhin Schulter 80% Besserung, Finger 50% Besserung mit Abschwellung von 8,2 cm linker Daumenumfang MCP Gelenk auf 6,6 cm. Sprunggelenk noch kein klarer Effekt. CRP innerhalb von 1 Woche von 20,3 mg/l auf 2,9 mg/l vermindert.

## Patentansprüche

1. Pharmazeutische Zusammensetzung enthaltend ein Corticosteroid zusammen mit Exosomen in Gegenwart oder Abwesenheit eines Cytokin-Antagonisten und/oder eines Wachstumsfaktors, wobei die Exosomen herstellbar sind durch ein Verfahren umfassend die Schritte: Bereitstellen einer Exosomen enthaltenden Blutprobe und vorzugsweise Aufkonzentrieren der Exosomen.

2. Pharmazeutische Zusammensetzung enthaltend Exosomen in Gegenwart oder Abwesenheit eines Cytokin-Antagonisten und/oder eines Wachstumsfaktors zur Verwendung in einer Kombinationstherapie zusammen mit einem Corticosteroid bei der Behandlung einer Krankheit, die ausgewählt aus der Gruppe bestehend aus Gelenkerkrankungen wie Arthrose, Arthritis, Gelenkentzündung und entzündlicher Knorpelverlust, degenerative Wirbelsäulenerkrankungen, Gelenkschmerzen und Autoimmunerkrankungen ist, wobei die Krankheit von rheumatoider Arthritis verschieden ist und die Exosomen herstellbar sind durch ein Verfahren umfassend die Schritte: Bereitstellen einer Exosomen enthaltenden Blutprobe und vorzugsweise Aufkonzentrieren der Exosomen.

3. Pharmazeutische Zusammensetzung enthaltend ein Corticosteroid in Gegenwart oder Abwesenheit eines Cytokin-Antagonisten und/oder eines Wachstumsfaktors zur Verwendung in einer Kombinationstherapie zusammen mit Exosomen bei der Behandlung einer Krankheit, die ausgewählt aus der Gruppe bestehend aus Gelenkerkrankungen wie Arthrose, Arthritis, Gelenkentzündung und entzündlicher Knorpelverlust, degenerative Wirbelsäulenerkrankungen, Gelenkschmerzen und Autoimmunerkrankungen ist, wobei die Krankheit von rheumatoider Arthritis verschieden ist und die Exosomen aus einer Blutprobe gewonnen wurden.

4. Pharmazeutische Zusammensetzung enthaltend einen Cytokin-Antagonisten und/oder einen Wachstumsfaktor zur Verwendung in einer Kombinationstherapie zusammen mit einem Corticosteroid und Exosomen bei der Behandlung einer Krankheit, die ausgewählt aus der Gruppe bestehend aus Gelenkerkrankungen wie Arthrose, Arthritis, Gelenkentzündung und entzündlicher Knorpelverlust, degenerative Wirbelsäulenerkrankungen, Gelenkschmerzen und Autoimmunerkrankungen ist, wobei die Krankheit von rheumatoider Arthritis verschieden ist und die Exosomen aus einer Blutprobe gewonnen wurden.

5. Kit enthaltend (i) eine pharmazeutische Zusammensetzung enthaltend Exosomen in Gegenwart oder Abwesenheit eines Cytokin-Antagonisten und/oder eines Wachstumsfaktors und (ii) eine pharmazeutische Zusammensetzung enthaltend ein Corticosteroid in Gegenwart oder Abwesenheit eines Cytokin-Antagonisten und/oder eines Wachstumsfaktors, wobei die Exosomen herstellbar sind durch ein Verfahren umfassend die Schritte: Bereitstellen einer Exosomen enthaltenden Blutprobe und vorzugsweise Aufkonzentrieren der Exosomen.

6. Pharmazeutische Zusammensetzung zur Verwendung gemäß einem der Ansprüche 2 oder 3 oder Kit gemäß Anspruch 5, wobei die pharmazeutische(n) Zusammensetzung(en) für eine simultane oder eine sequenzielle Verabreichung der Exosomen und des Corticosteroids bestimmt ist (sind).

7. Pharmazeutische Zusammensetzung, pharmazeutische Zusammensetzung zur Verwendung, oder Kit gemäß einem der Ansprüche 1 bis 6, wobei das Corticosteroid
(a) ein Glucocorticoid wie beispielsweise Cortison, Hydrocortison, Prednison, Prednisolon, Cloprednol, Deflazacort, Fluocortin, Triamcinolon, Dexamethason, Methylprednisolon, Fluprednisolon, Clocortolon, Clobetason, Alclomethason, Flumethason, Fluopredniden, Fluorandrenolon, Betamethason, Beclomethason, Fluocortolon, Mometason, Fluticason, Halomethason, Fluocinolon, Diflorason, Desoximethason, Fluocinonid, Amcinonid, Halcinonid, Diflucortolon, Clobetasol, Paramethason;
(b) ein Mineralocorticoid wie beispielsweise Aldosteron, Desoxycorticosteron und Fludrocortison; oder
(c) ein Androgen wie beispielsweise Dehydroepiandrosteron (DHEA) und Estrogene;
oder ein Salz, Ester oder Prodrug davon ist.

8. Pharmazeutische Zusammensetzung, pharmazeutische Zusammensetzung zur Verwendung, oder Kit gemäß einem der Ansprüche 1, 3, 5, 6 oder 7, wobei das Corticosteroid in einer Konzentration von 1 bis 80 mg/Dosis in der Corticosteroid-enthaltenden pharmazeutischen Zusammensetzung vorliegt.

9. Pharmazeutische Zusammensetzung zur Verwendung, oder Kit gemäß einem der Ansprüche 3, 4, 6, 7 oder 8, wobei die Exosomen bezüglich des zu behandelnden Patienten autolog oder allogen sind.

10. Pharmazeutische Zusammensetzung oder Kit gemäß einem der Ansprüche 1 oder 5 bis 9 zur Verwendung bei der Behandlung von Gelenkerkrankungen wie Arthrose, Arthritis, Gelenkentzündung und entzündlichem Knorpelverlust, degenerativen Wirbelsäulenerkrankungen, und/oder Gelenkschmerzen.

11. Pharmazeutische Zusammensetzung oder Kit zur Verwendung gemäß einem der Ansprüche 2 bis 4 oder 6 bis 10, wobei die Arthrose eine aktivierte Arthrose oder eine entzündliche Arthrose ist.

12. Pharmazeutische Zusammensetzung oder Kit zur Verwendung gemäß einem der Ansprüche 2 bis 4 oder 6 bis 10, wobei die Arthritis eine infektionsbedingte Arthritis wie bakterielle Arthritis oder eine nicht-infektionsbedingte Arthritis ist.

13. Pharmazeutische Zusammensetzung oder Kit zur Verwendung gemäß einem der Ansprüche 2 bis 4 oder 6 bis 10, wobei die degenerative Wirbelsäulenerkrankung ein Bandscheibenvorfall ist.

14. Pharmazeutische Zusammensetzung oder Kit gemäß einem der Ansprüche 1 oder 5 bis 9 zur Verwendung bei der Behandlung einer Autoimmunerkrankung.

15. Pharmazeutische Zusammensetzung oder Kit zur Verwendung gemäß einem der Ansprüche 2 bis 4, 6 bis 9 oder 14, wobei die Autoimmunerkrankung Neurodermitis oder alopecia areata ist.

16. Pharmazeutische Zusammensetzung, pharmazeutische Zusammensetzung zur Verwendung, oder Kit gemäß einem der Ansprüche 1 bis 15, wobei die pharmazeutische(n) Zusammensetzung(en) für eine lokale Verabreichung geeignet ist (sind).

17. Pharmazeutische Zusammensetzung, pharmazeutische Zusammensetzung zur Verwendung, oder Kit gemäß Anspruch 16, wobei die lokale Verabreichung ausgewählt ist aus der Gruppe bestehend aus Injektion in die betroffene Körperregion, insbesondere in das betroffene Gelenk, an die betroffene Nervenwurzel oder in die betroffene Bandscheibe, oder in die lokale Umgebung hiervon; intraartikuläre Injektion; und topische Verabreichung.

18. Pharmazeutische Zusammensetzung, pharmazeutische Zusammensetzung zur Verwendung, oder Kit gemäß einem der Ansprüche 1 bis 17, wobei die pharmazeutische(n) Zusammensetzung(en) ferner einen Träger und/oder ein Exzipienten enthält (enthalten).

19. Pharmazeutische Zusammensetzung, pharmazeutische Zusammensetzung zur Verwendung, oder Kit gemäß einem der Ansprüche 1 bis 18, wobei ein Cytokin-Antagonist anwesend ist und der Cytokin-Antagonist ausgewählt ist aus der Gruppe bestehend aus Interleukin-Antagonisten, insbesondere IL-1-Antagonisten wie IL-1Ra, Tumornekrosefaktor (TNF)-Antagonisten, insbesondere ein TNF-α-Antagonist wie ein Anti-TNF-α-Antikörper, Interferon-Antagonisten, und Chemokin-Antagonisten.

20. Pharmazeutische Zusammensetzung, pharmazeutische Zusammensetzung zur Verwendung, oder Kit gemäß Anspruch 19, wobei der Cytokin-Antagonist natürlich vorkommendes oder rekombinantes IL-1 Ra-Protein, insbesondere Orthokin oder Anakinra ist.

21. Pharmazeutische Zusammensetzung, pharmazeutische Zusammensetzung zur Verwendung, oder Kit gemäß einem der Ansprüche 19 oder 20, wobei der Cytokin-Antagonist in einer Konzentration von 0,5 bis 150 mg/Dosis in der Cytokin-Antagonist-enthaltenden pharmazeutischen Zusammensetzung vorliegt.

22. Pharmazeutische Zusammensetzung, pharmazeutische Zusammensetzung zur Verwendung, oder Kit gemäß einem der Ansprüche 1 bis 21, wobei ein Wachstumsfaktor anwesend ist und der Wachstumsfaktor ausgewählt ist aus der Gruppe bestehend aus TGF-β, IGF, BMP, HGF und VEGF.

23. Verwendung von Exosomen in Gegenwart oder Abwesenheit eines Cytokin-Antagonisten und/oder eines Wachstumsfaktors zur Herstellung einer pharmazeutischen Zusammensetzung zur Verwendung in einer Kombinationstherapie zusammen mit einem Corticosteroid bei der Behandlung einer Krankheit, die ausgewählt aus der Gruppe bestehend aus Gelenkerkrankungen wie Arthrose, Arthritis, Gelenkentzündung und entzündlicher Knorpelverlust, degenerative Wirbelsäulenerkrankungen, Gelenkschmerzen und Autoimmunerkrankungen ist, wobei die Krankheit von rheumatoider Arthritis verschieden ist und die Exosomen herstellbar sind durch ein Verfahren umfassend die Schritte: Bereitstellen einer Exosomen enthaltenden Blutprobe und vorzugsweise Aufkonzentrieren der Exosomen.

24. Verwendung eines Corticosteroids in Gegenwart oder Abwesenheit eines Cytokin-Antagonisten und/oder eines Wachstumsfaktors zur Herstellung einer pharmazeutischen Zusammensetzung zur Verwendung in einer Kombinationstherapie zusammen mit Exosomen bei der Behandlung einer Krankheit, die ausgewählt aus der Gruppe bestehend aus Gelenkerkrankungen wie Arthrose, Arthritis, Gelenkentzündung und entzündlicher Knorpelverlust, degenerative Wirbelsäulenerkrankungen, Gelenkschmerzen und Autoimmunerkrankungen ist, wobei die Krankheit von rheumatoider Arthritis verschieden ist und die Exosomen aus einer Blutprobe gewonnen wurden.

25. Verfahren zur Herstellung einer ein Corticosteroid und Exosomen enthaltenden pharmazeutischen Zusammensetzung, umfassend die Schritte: Bereitstellen einer Exosomen enthaltenden Blutprobe, vorzugsweise Aufkonzentrieren der Exosomen und Vermischen mit einem Corticosteroid.

## Claims

1. Pharmaceutical composition containing a corticosteroid together with exosomes in the presence or absence of a cytokine antagonist and/or a growth factor, wherein the exosomes can be manufactured by a method comprising the steps: Provision of a blood sample containing exosomes and preferably increasing the concentration of the exosomes.

2. Pharmaceutical composition containing exosomes in the presence or absence of a cytokine antagonist and/or a growth factor for use in a combination therapy together with a corticosteroid in the treatment of a disease, which is selected from the group consisting of joint diseases, such as arthrosis, arthritis, joint inflammation and inflammatory cartilage loss, degenerative vertebral diseases, joint pains and autoimmune diseases, wherein the disease is different from rheumatoid arthritis, and the exosomes can be manufactured by a method comprising the steps: Provision of a blood sample containing exosomes and preferably increasing the concentration of the exosomes.

3. Pharmaceutical composition containing a corticosteroid in the presence or absence of a cytokine antagonist and/or a growth factor for use in a combination therapy together with exosomes in the treatment of a disease, which is selected from the group consisting of joint diseases, such as arthrosis, arthritis, joint inflammation and inflammatory cartilage loss, degenerative vertebral diseases, joint pains and autoimmune diseases, wherein the disease is different from rheumatoid arthritis, and the exosomes have been obtained from a blood sample.

4. Pharmaceutical composition containing a cytokine antagonist and/or a growth factor for use in a combination therapy together with a corticosteroid and exosomes in the treatment of a disease, which is selected from the group consisting of joint diseases, such as arthrosis, arthritis, joint inflammation and inflammatory cartilage loss, degenerative vertebral diseases, joint pains and autoimmune diseases, wherein the disease is different from rheumatoid arthritis, and the exosomes have been obtained from a blood sample.

5. Kit containing (i) a pharmaceutical composition containing exosomes in the presence or absence of a cytokine antagonist and/or a growth factor and (ii) a pharmaceutical composition containing a corticosteroid in the presence or absence of a cytokine antagonist and/or a growth factor, wherein the exosomes can be manufactured by a method comprising the steps: Provision of a blood sample containing exosomes and preferably increasing the concentration of the exosomes.

6. Pharmaceutical composition for use according to one of claims 2 or 3 or kit according to claim 5, wherein the pharmaceutical composition(s) is (are) determined for a simultaneous or sequential application of the exosomes and of the corticosteroid.

7. Pharmaceutical composition, pharmaceutical composition for use, or kit according to one of claims 1 to 6, wherein the corticosteroid is
(a) a glucocorticoid, such as cortisone, hydrocortisone, prednisone, prednisolone, cloprednole, deflazacort, fluocortin, triamcinolone, dexamethasone, methylprednisolone, fluprednisolone, clocortolone, clobetasone, alclomethasone, flumethasone, fluprednidene, flurandrenolone, betamethasone, beclomethasone, fluocortolone, mometasone, fluticasone, halomethasone, fluocinolone, diflorasone, desoximethasone, fluocinonide, amcinonide, halcinonide, diflucortolone, clobetasol, paramethasone;
(b) a mineralocorticoid, such as aldosterone, desoxycorticosterone and fludrocortisone; or
(c) an androgen, such as dehydroepiandrosterone (DHEA) and estrogens; or a salt, ester or prodrug thereof.

8. Pharmaceutical composition, pharmaceutical composition for use, or kit according to one of claims 1, 3, 5, 6 or 7, wherein the corticosteroid is present in a concentration from 1 to 80 mg/dose in the corticosteroid-containing pharmaceutical composition.

9. Pharmaceutical composition for use, or kit according to one of claims 3, 4, 6, 7 or 8, wherein the exosomes are autologous or allogenic with reference to the patient to be treated.

10. Pharmaceutical composition or kit according to one of claims 1 or 5 to 9 for use in the treatment of joint diseases, such as arthrosis, arthritis, joint inflammation and inflammatory cartilage loss, degenerative vertebral diseases and/or joint pain.

11. Pharmaceutical composition or kit for use according to one of claims 2 to 4 or 6 to 10, wherein the arthrosis is an activated arthrosis or an inflammatory arthrosis.

12. Pharmaceutical composition or kit for use according to one of claims 2 to 4 or 6 to 10, wherein the arthritis is an infectious arthritis such as bacterial arthritis or a non-infectious arthritis.

13. Pharmaceutical composition or kit for use according to one of claims 2 to 4 or 6 to 10, wherein the degenerative vertebral disease is a vertebral disc prolapse.

14. Pharmaceutical composition or kit according to one of claims 1 or 5 to 9 for use in the treatment of an autoimmune disease.

15. Pharmaceutical composition or kit for use according to one of claims 2 to 4, 6 to 9 to 14, wherein the autoimmune disease is neurodermatitis or alopecia areata.

16. Pharmaceutical composition, pharmaceutical composition for use, or kit according to one of claims 1 to 15, wherein the pharmaceutical composition(s) is (are) suitable for a local application.

17. Pharmaceutical composition, pharmaceutical composition for use or kit according to claim 16, wherein the local application is selected from the group consisting of injection into the affected body region, especially into the affected joint, into the affected nerve root or into the affected vertebral disc, or into the local environment thereof; intra-articular injection; and topical application.

18. Pharmaceutical composition, pharmaceutical composition for use, or kit according to one of claims 1 to 17, wherein the pharmaceutical composition(s) further contain(s) a carrier and/or an excipient.

19. Pharmaceutical composition, pharmaceutical composition for use, or kit according to one of claims 1 to 18, wherein a cytokine antagonist is present, and the cytokine antagonist is selected from the group consisting of interleukin antagonists, especially IL-1-antagonists such as IL-1Ra, tumour necrosis factor (TNF) antagonists, especially a TNF-α- antagonist such as an anti-TNF-α-antibody, interferon antagonists, and chemokine antagonists.

20. Pharmaceutical composition, pharmaceutical composition for use, or kit according to claim 19, wherein the cytokine antagonist is naturally occurring or recombinant IL-1Ra protein, especially orthokine or anakinra.

21. Pharmaceutical composition, pharmaceutical composition for use, or kit according to one of claims 19 or 20, wherein the cytokine antagonist is present in a concentration from 0.5 to 150 mg/dose in the cytokine-antagonist-containing pharmaceutical composition.

22. Pharmaceutical composition, pharmaceutical composition for use, or kit according to one of claims 1 to 21, wherein a growth factor is present, and the growth factor is selected from the group consisting of TGF-β, IGF, BMP, HGF and VEGF.

23. Use of exosomes in the presence or absence of a cytokine antagonist and/or a growth factor for the manufacture of a pharmaceutical composition for use in a combination therapy together with a corticosteroid in the treatment of a disease, which is selected from the group consisting of joint diseases, such as arthrosis, arthritis, joint inflammation and inflammatory cartilage loss, degenerative vertebral diseases, joint pains and autoimmune diseases, wherein the disease is different from rheumatoid arthritis, and the exosomes can be manufactured by a method comprising the steps: Provision of a blood sample containing exosomes and preferably increasing the concentration of the exosomes.

24. Use of a corticosteroid in the presence or absence of a cytokine antagonist and/or a growth factor for the manufacture of a pharmaceutical composition for use in a combination therapy together with exosomes in the treatment of a disease, which is selected from the group consisting of joint diseases, such as arthrosis, arthritis, joint inflammation and inflammatory cartilage loss, degenerative vertebral diseases, joint pains and autoimmune diseases, wherein the disease is different from rheumatoid arthritis, and the exosomes have been obtained from a blood sample.

25. Method for the manufacture of a corticosteroid and pharmaceutical composition containing exosomes, comprising the steps: Provision of a blood sample containing exosomes, preferably increasing the concentration of the exosomes and mixing with a corticosteroid.

## Revendications

1. Composition pharmaceutique contenant un corticoïde conjointement avec des exosomes en présence ou en l'absence d'un antagoniste de cytokine et/ou d'un facteur de croissance, dans laquelle les exosomes peuvent être produits par un procédé comportant les étapes de : préparation d'un échantillon sanguin contenant des exosomes et de préférence concentration des exosomes.

2. Composition pharmaceutique contenant des exosomes en présence ou en l'absence d'un antagoniste de cytokine et/ou d'un facteur de croissance pour utilisation dans un traitement combiné conjointement avec un corticoïde pour le traitement d'une maladie, qui est choisie parmi le groupe constitué de maladies articulaires telles que arthrose, arthrite, inflammation articulaire et destruction inflammatoire du cartilage, maladies dégénératives de la colonne vertébrale, douleurs articulaires et maladies auto-immunes, la maladie étant différente de la polyarthrite rhumatoïde et les exosomes pouvant être préparés par un procédé comprenant les étapes de : préparation d'un échantillon sanguin contenant des exosomes et de préférence concentration des exosomes.

3. Composition pharmaceutique contenant un corticoïde en présence ou en l'absence d'un antagoniste de cytokine et/ou d'un facteur de croissance pour utilisation dans un traitement combiné conjointement avec des exosomes pour le traitement d'une maladie, qui est choisie parmi le groupe constitué de maladies articulaires telles que arthrose, arthrite, inflammation articulaire et destruction inflammatoire du cartilage, maladies dégénératives de la colonne vertébrale, douleurs articulaires et maladies auto-immunes, la maladie étant différente de la polyarthrite rhumatoïde et les exosomes étant préparés à partir d'un échantillon sanguin.

4. Composition pharmaceutique contenant un antagoniste de cytokine et/ou un facteur de croissance pour utilisation dans un traitement combiné conjointement avec un corticoïde et des exosomes pour le traitement d'une maladie, qui est choisie parmi le groupe constitué de maladies articulaires telles que arthrose, arthrite, inflammation articulaire et destruction inflammatoire du cartilage, maladies dégénératives de la colonne vertébrale, douleurs articulaires et maladies auto-immunes, la maladie étant différente de la polyarthrite rhumatoïde et les exosomes étant préparés à partir d'un échantillon sanguin.

5. Trousse contenant (i) une composition pharmaceutique contenant des exosomes en présence ou en l'absence d'un antagoniste de cytokine et/ou d'un facteur de croissance et (ii) une composition pharmaceutique contenant un corticoïde en présence ou en l'absence d'un antagoniste de cytokine et/ou d'un facteur de croissance, dans laquelle les exosomes peuvent être produits par un procédé comportant les étapes de : préparation d'un échantillon sanguin contenant des exosomes et de préférence concentration des exosomes.

6. Composition pharmaceutique pour utilisation selon l'une des revendications 2 ou 3 ou trousse selon la revendication 5, la ou les compositions pharmaceutiques étant destinées à une administration simultanée ou séquentielle des exosomes et du corticoïde.

7. Composition pharmaceutique, composition pharmaceutique pour utilisation, ou trousse selon l'une des revendications 1 à 6, dans laquelle le corticoïde est
(a) un glucocorticoïde comme par exemple, cortisone, hydrocortisone, prednisone, prednisolone, cloprednol, déflazacort, fluocortine, triamcinolone, dexaméthasone, méthylprednisolone, fluprednisolone, clocortolone, clobétasone, alclométhasone, fluméthasone, fluprednidène, flurandrénolone, bêtaméthasone, béclométhasone, fluocortolone, mométasone, fluticasone, halométhasone, fluocinolone, diflorasone, désoxyméthasone, fluocinonide, amcinonide, halcinonide, diflucortolone, clobétasol, paraméthasone ;
(b) un minéralocorticoïde tel que par exemple l'aldostérone, la désoxycorticostérone et la fludrocortisone ; ou
(c) un androgène tel que par exemple la déhydroépiandrostérone (DHEA) et des oestrogènes ;
ou un de leurs sels, esters ou pro-médicament.

8. Composition pharmaceutique, composition pharmaceutique pour utilisation, ou trousse selon l'une des revendications 1, 3, 5, 6 ou 7, le corticoïde étant présent à une concentration de 1 à 80 mg/dose dans la composition pharmaceutique contenant le corticoïde.

9. Composition pharmaceutique pour utilisation, ou trousse selon l'une des revendications 3, 4, 6, 7 ou 8, dans laquelle les exosomes sont autologues ou allogènes par rapport au patient à traiter.

10. Composition pharmaceutique ou trousse selon l'une des revendications 1 ou 5 à 9 pour utilisation dans le traitement de maladies articulaires telles que arthrose, arthrite, inflammation articulaire et destruction inflammatoire du cartilage, de maladies dégénératives de la colonne vertébrale, et/ou de douleurs articulaires.

11. Composition pharmaceutique ou trousse pour utilisation selon l'une des revendications 2 à 4 ou 6 à 10, l'arthrose étant une arthrose activée ou une arthrose inflammatoire.

12. Composition pharmaceutique ou trousse pour utilisation selon l'une des revendications 2 à 4 ou 6 à 10, l'arthrite étant une arthrite infectieuse telle qu'une arthrite bactérienne ou une arthrite non infectieuse.

13. Composition pharmaceutique ou trousse pour utilisation selon l'une des revendications 2 à 4 ou 6 à 10, la maladie dégénérative de la colonne vertébrale étant une hernie discale.

14. Composition pharmaceutique ou trousse selon l'une des revendications 1 ou 5 à 9 pour utilisation dans le traitement d'une maladie auto-immune.

15. Composition pharmaceutique ou trousse pour utilisation selon l'une des revendications 2 à 4, 6 à 9 ou 14, la maladie auto-immune étant la neurodermite ou l'alopécie aerata.

16. Composition pharmaceutique, composition pharmaceutique pour utilisation, ou trousse selon l'une des revendications 1 à 15, la ou les compositions pharmaceutiques convenant pour une administration locale.

17. Composition pharmaceutique, composition pharmaceutique pour utilisation, ou trousse selon la revendication 16, l'administration locale étant choisie parmi le groupe constitué d'injection dans la région corporelle concernée, en particulier dans l'articulation concernée, dans la racine nerveuse concernée ou dans le disque intervertébral concerné ou dans son environnement local ; injection intra-articulaire ; et administration topique.

18. Composition pharmaceutique, composition pharmaceutique pour utilisation, ou trousse selon l'une des revendications 1 à 17, la ou les compositions pharmaceutiques contenant en outre un véhicule et/ou un excipient.

19. Composition pharmaceutique, composition pharmaceutique pour utilisation, ou trousse selon l'une des revendications 1 à 18, dans laquelle est présent un antagoniste de cytokine et l'antagoniste de cytokine est choisi parmi le groupe constitué d'antagonistes d'interleukine, en particulier d'antagonistes d'IL-1 tels que IL-1Ra, d'antagonistes du facteur de nécrose tumorale (TNF), en particulier un antagoniste de TNF-α tel qu'un anticorps anti-TNF-α, d'antagonistes de l'interféron et d'antagonistes de chimiokine.

20. Composition pharmaceutique, composition pharmaceutique pour utilisation, ou trousse selon la revendication 19, dans laquelle l'antagoniste de cytokine est la protéine IL-1RA naturelle ou recombinante, en particulier l'orthokine ou l'anakinra.

21. Composition pharmaceutique, composition pharmaceutique pour utilisation, ou trousse selon l'une des revendications 19 ou 20, l'antagoniste de cytokine étant présent à une concentration de 0,5 à 150 mg/dose dans la composition pharmaceutique contenant l'antagoniste de cytokine.

22. Composition pharmaceutique, composition pharmaceutique pour utilisation, ou trousse selon l'une des revendications 1 à 21, dans laquelle un facteur de croissance est présent et le facteur de croissance est choisi parmi le groupe constitué de TGF-ß, IGF, BMP, HGF et VEGF.

23. Utilisation d'exosomes en présence ou en l'absence d'un antagoniste de cytokine et/ou d'un facteur de croissance pour la préparation d'une composition pharmaceutique pour utilisation dans un traitement combiné conjointement avec un corticoïde pour le traitement d'une maladie, qui est choisie parmi le groupe constitué de maladies articulaires telles que arthrose, arthrite, inflammation articulaire et destruction inflammatoire du cartilage, maladies dégénératives de la colonne vertébrale, douleurs articulaires et maladies auto-immunes, la maladie étant différente de la polyarthrite rhumatoïde et les exosomes pouvant être préparés par un procédé comprenant les étapes de : préparation d'un échantillon sanguin contenant des exosomes et de préférence concentration des exosomes.

24. Utilisation d'un corticoïde en présence ou en l'absence d'un antagoniste de cytokine et/ou d'un facteur de croissance pour la préparation d'une composition pharmaceutique pour utilisation dans un traitement combiné conjointement avec des exosomes pour le traitement d'une maladie, qui est choisie parmi le groupe constitué de maladies articulaires telles que arthrose, arthrite, inflammation articulaire et destruction inflammatoire du cartilage, maladies dégénératives de la colonne vertébrale, douleurs articulaires et maladies auto-immunes, la maladie étant différente du de la polyarthrite rhumatoïde et les exosomes étant préparés à partir d'un échantillon sanguin.

25. Procédé de préparation d'une composition pharmaceutique contenant un corticoïde et des exosomes, comportant les étapes de : préparation d'un échantillon sanguin contenant des exosomes, de préférence concentration des exosomes et mélange avec un corticoïde.
